# NEW EUROPEAN PATENT SPECIFICATION

(11) **EP 2 124 832 B2**
(45) Date of publication and mention of the opposition decision: **19.07.2017**
(45) Mention of the grant of the patent: 08.08.2012
(21) Application number: 07848644.6
(22) Date of filing: 20.12.2007
(51) Int. Cl.: A61F 2/42, A61B 17/15

(54) **IMPROVEMENTS IN AND RELATING TO AN ANKLE PROSTHESIS**
SPRUNGGELENKPROTHESE
PERFECTIONNEMENTS À ET APPARENTÉS À UNE PROTHÈSE DE CHEVILLE

(30) Priority: 23.12.2006 GB 0625925
(43) Date of publication of application: 02.12.2009
(73) Proprietor: Corin Limited, The Corinium Centre Cirencester GL7 1YJ (GB)
(72) Inventor: COLLINS, Simon Nicholas, Tetbury Gloucestershire GL8 8NT (GB); WINSON, Ian Geoffrey, Nr Bristol BS32 4L (GB); FLETCHER, David Mark, Tetbury Gloucestershire GL8 8TF (GB); SUNGU, Mevlut, 66121 Saarbrucken (DE)
(74) Representative: Hocking, Adrian Niall
(86) International application number: PCT/GB2007/004915
(87) International publication number: WO 2008/078082

(56) References cited:
- EP-A- 1 393 696
- WO-A-00/69373
- WO-A-2007/084846
- WO-A1-03/075802
- DE-U1- 8 812 806
- FR-A1- 2 676 917
- JP-A- 2002 143 192
- JP-U- 5 041 510
- US-A- 3 896 503
- US-A- 3 987 500
- US-A1- 2002 055 744
- US-A1- 2004 002 768
- US-A1- 2005 004 676
- US-A1- 2005 049 711
- US-A1- 2005 171 608
- US-A1- 2006 020 345
- US-A1- 2006 142 870
- US-A1- 2006 229 730
- US-B1- 6 409 767

## Description

The present invention relates to a talar component of an ankle prosthesis,

Existing talar components for ankle prosthesis have been troublesome to introduce, typically comprising large block-shaped projections formed centrally on an inferior surface. The projection stabilises the talar component, but does require significant amounts of the talus to be removed to allow introduction. WO 00/69373 and US 2005/004676 both disclose ankle prosthesis devices, which consist of talar components with stabilising elements protruding from more than one surface portion.

The talar component of the present invention thus seeks to provide a solution to this problem, whereby stability is maintained, but introduction is simplified.

Heretobefore, it has been the responsibility of the surgeon to form angled surfaces on a talus. It is known to perform this simply by eye. This has resulted in trial and error resecting of the bone intraoperatively, and consequently an operation can take longer than anticipated while the correct angle to accept a talar component is formed.

The talar resecting jig, which falls outside the scope of the present invention, seeks to provide a solution to this problem.

Previously, it has also been solely down to the ability of a surgeon to judge an amount of bone to remove from both a distal end of a patient's tibia and a superior surface of the talus in order to accept an ankle prosthesis comprising a tibial component, a talar component and a bearing interposed therebetween, It is, however, important that ankle ligament tension is maintained to ensure that post-operative stability and function is optimised. If too much bone is removed, increased joint laxity may occur leading to potential instability and/or subluxation. If too little bone is removed, the joint would be tight and the range of motion may be compromised.

The ankle resecting jig, which falls outside of the present invention, seeks to provide a solution to this problem, thus allowing an accurate amount of resection to occur so that correct ligament tension is maintained.

According to the invention, there is provided a talar component comprising a superior surface profiled to receive a talar bearing, and a concave inferior surface, the inferior surface having three planar contiguous surface portions which are angled relative to each other, and each surface portion extending across or substantially across a lateral extent of the talar component, wherein one or more wedging stabilisation elements extend from one or more of the surface portions in a direction from the superior surface to the inferior surface, being characterised by the or each stabilisation element extending from only an anterior said surface portion which is at or closest to the anterior edge of the component.

Preferable and/or optional features of the first aspect of the invention are set forth in claims 2 to 5, inclusive.

The invention will now be more particularly described, by way of example only, with reference to the accompanying drawings, in which :
Figure 1 is a perspective view of one embodiment of an ankle resecting jig, not forming part of the invention;
Figure 2 is an elevational view of one side of the jig shown in Figure 1, in the direction of arrow A;
Figure 3 is an elevational view of another side of the jig, shown in Figure 1, in the direction of arrow B;
Figure 4 is a perspective view of one embodiment of a talar resecting jig, not in accordance with the invention and showing a head of a surgical cutting device located therein;
Figure 5 is a side elevational view of the jig shown in Figure 4, in the direction of arrow C;
Figure 6 is a side elevational view of the jig shown in Figure 4, in the direction of arrow D;
Figure 7 is a perspective view from above of one embodiment of a talar component, in accordance with the present invention;
Figure 8 is a perspective view from below of the talar component;
Figure 9 is a side view of the talar component; and
Figure 10 is a view from the posterior edge of the talar component.

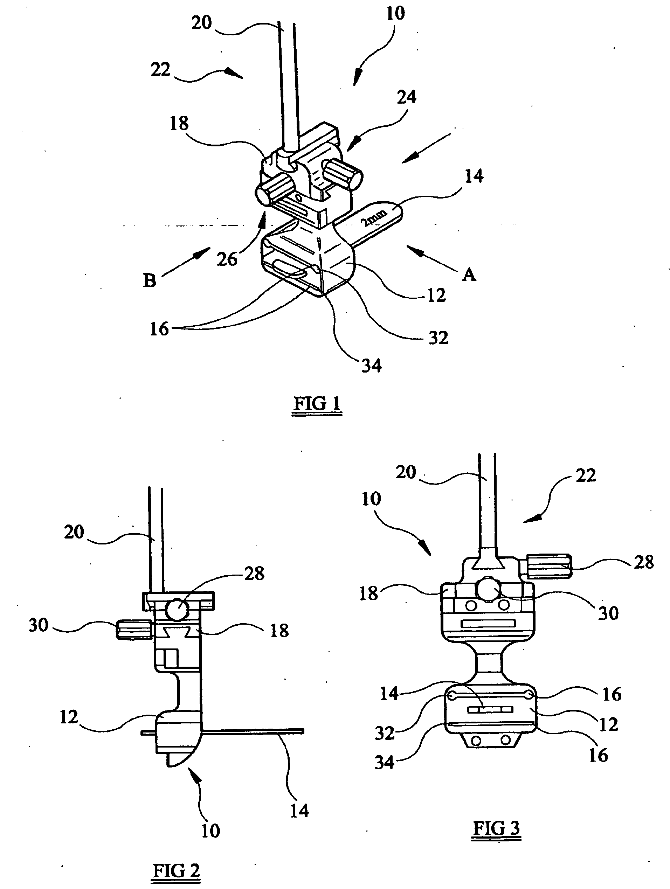

Referring firstly to Figures 1 to 3 of the drawings, there is shown an ankle resecting jig 10 for an ankle prosthesis, the jig comprising a jig body 12, a spacer element 14 which projects from the jig body 12, and a cutting guide 16 for receiving and guiding a cutting device (not shown). The ankle resecting jig falls outside the scope of the present invention.

Typical materials of the jig body are surgical grade stainless steels or high performance plastics and/or ceramics, and for the spacer element are Ultra High Weight Polyethylene or other suitable surgical plastics.

The jig body 12 is attachable at one end to a movable saddle 18 which, along with an elongate support element 20, forms part of a tibial guide 22. The saddle 18 forms part of two transverse adjustment mechanisms 24, 26, controlled via two rotatable control knobs 28, 30, respectively, which allow movement of the jig body 12 in two mutually perpendicular transverse directions normal to the longitudinal extent of the tibial guide 22.

Adjustment of the jig body 12 along the longitudinal extent of the elongate support element 20 of the tibial guide 22 is also possible. Typically, a ratchet mechanism or clamp mechanism is utilised.

The spacer element 14 is push-fit engagable with, and removable from, the jig body 12. The spacer element 14 has a predetermined thickness, and is selectable from a plurality of spacer elements 14 having differing thicknesses. The spacer element 14 is generally tongue shaped, and extends perpendicularly relative to the longitudinal extent of the tibial guide 22.

The cutting guide 16 includes two guide slots 32, 34, spaced in the longitudinal direction of the tibial guide 22, and positioned above and below the spacer element 14, respectively. The upper guide slot 32, in use nearest to the tibial guide 22, is a tibial slot for receiving a cutting device for resecting a distal end of a tibia, and the lower guide slot 32, in use furthest from the tibial guide 22, is a talus slot for receiving a cutting device for resecting a superior surface of a talus.

The tibial slot 32 and the talus slot 34 extend through the jig body 12 in parallel with each other, and in parallel with the spacer element 14. However, it is possible that the tibial slot and the talus slot need not be parallel, and/or the relative positioning of the two slots 32, 34 could be adjustable. In this latter case, one or both slots 32, 34 can have mechanically adjustable surfaces, as easily envisaged by one skilled in the field.

In use and with the ankle of the patient pre-prepared and at or substantially at 90 degrees to the tibia, the elongate support element 20 of the tibial guide 22 is pinned to the anterior surface of the patient's tibia, and the jig body 12, with selected spacer element 14, is aligned with the ankle joint via the adjustment mechanisms 24, 26 so that the spacer element 14 is interposed between the distal end of the tibia and the superior surface of the talus.

With the spacer element 14 interposed, the tension of the ankle ligaments is checked. If the ankle joint feels tight, then the spacer element 14 is too thick. The spacer element 14 is thus removed and replaced with a thinner spacer element 14. Conversely, if the ankle joint feels lax, then the spacer element 14 is replaced with one having a greater thickness.

The thickness of the spacer element 14 sets the distance between the distal end of the tibia and the superior surface of the talus.

Once the correct spacing of the tibia and the talus has been determined, via the spacer element 14, an oscillating saw or any other suitable cutting device can be inserted through the tibial slot 32, and the distal end of the tibia is resected by the correct amount to receive a tibial plate of a tibial component of an ankle prosthesis.

The oscillating saw or other cutting device is then inserted through the talus slot 34, and the superior surface of the patient's talus is resected by the correct amount to receive a talar plate of a talar component.

Once the talar component and the tibial component have been introduced and fixed in place, a sliding talar bearing is introduced between the tibial component and the talar component. A thickness of a talar plate of the talar component, a tibial plate of the tibial component, and the talar bearing matches or is close to a thickness of the selected spacer element 14 and the bone removed.

With the ankle prosthesis assembled and in place, a working thickness of the ankle prosthesis, being a distance between the tibial abutment surface of the tibial platform of the tibial component and a talar abutment surface of the talar platform of the talar component, thus corresponds to a correctly spaced distance between the resected surfaces of the tibia and the talus. Consequently, correct tension of the ligaments and thus correct mobility of the foot is restored.

It is feasible that the jig body can be attached to the patient's leg or foot by means other than a tibial guide, such as by a clamp or pins.

One or more spacer elements can be used to check spacing between the tibia and the talus prior to location of the jig body on the patient.

The spacer element can be a mechanical spacer device with moveable platforms or arms, instead of a plurality of interchangeable fixed-thickness devices. In this case, the spacer element can form part of or be permanently attached to the jig body.

The cutting guide can utilise only one slot. For example, the jig body could be invertible.

The slot or slots of the cutting guide may be dispensed with in favour of a guiding surface.

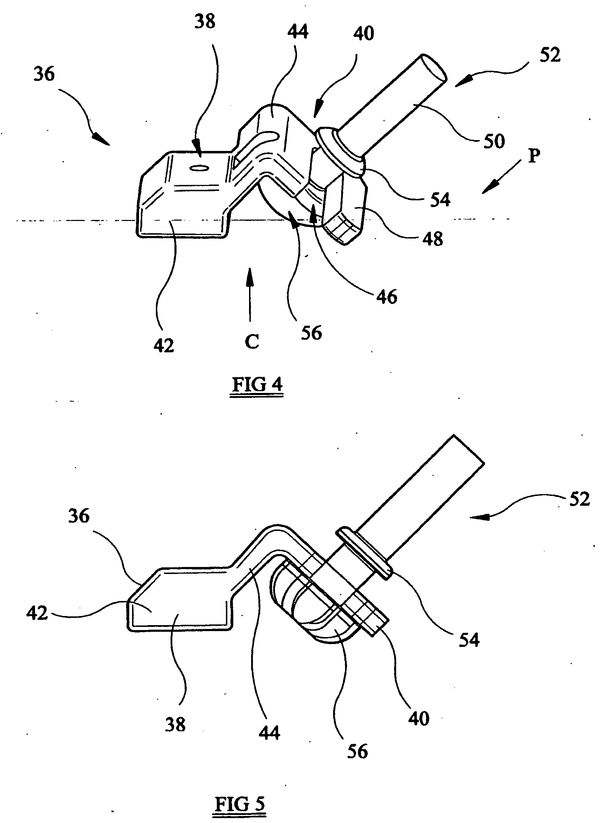

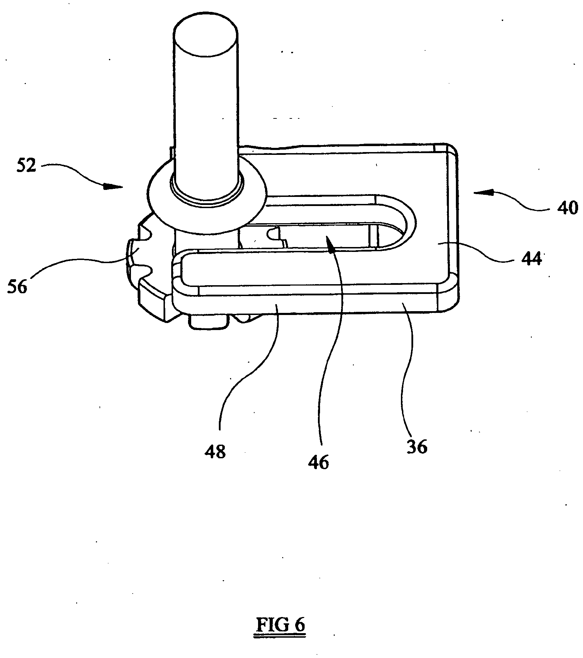

Referring now to Figures 4 to 6, there is shown a talus resecting jig 36 for forming a superior anterior surface on a talus at an angle to a longitudinal extent of a tibia, the jig comprising a support element 38, and a guide element 40 which is cranked relative to the support element 38. The talus resecting jig falls outside the scope of the present invention.

Typical materials of the jig 36 are surgical grade stainless steels or high performance plastics and/or ceramics.

The support element 38 includes an interposable block-shaped portion 42, and a cranked arm portion 44 which extends at an angle from an upper anterior edge of the interposable portion 42. The guide element 40 extends perpendicularly or substantially perpendicularly from an edge of the arm portion 44 which is remote from the interposable portion 42, such that the guide element 40 forms an obtuse included angle with a plane of the inferior surface of the interposable portion 42. A general overall shape of the jig 36 is thus one of a dog-leg.

The guide element 40 includes a guide slot 46 which extends transversely, and preferably laterally, partway across the guide element 40. The guide slot 46 originates at one edge of the guide element 40, and terminates at a position spaced from an opposing side edge. As such, the guide slot 46 is spaced from, and extends in parallel or substantially parallel with, the anterior edge 48 of the guide element 40.

The guide slot 46 is suitably dimensioned to receive a shaft 50 of a rotatable cutting device 52, such as a surgical cutting reamer and/or /burr, or a specialised anterior talus cutter. As shown in the drawings, the cutter 52 has a rotatable shaft 50, a collar 54 defining a stop, and a rotatable grinding or cutting head 56.

The guide slot 46 is sufficiently spaced from the arm portion 44 so that, when the cutting device 52 is moved along the guide slot 46, the head 56 of the cutting device can travel parallel or substantially parallel to an inferior surface of the guide element 40 without hindrance or obstruction. If using the talus cutter, the collar 54 aids location. Although, in the present case, the collar 54 is fixed, the collar can be movable axially along the shaft to bear on a surface of the arm portion to help maintain the cutting head 56 in place.

The following description of the talus resecting jig 36 in use assumes that the foot of the patient has no or substantially no flexion, and thus extends at or substantially at right angles to the tibia.

Once a superior surface of a patient's talus has been resected normally to a longitudinal extent of the tibia, for example, through the use of the ankle resecting jig 10 described above, the talus resecting jig 36 is used to form an angled superior anterior surface on the talus in readiness for accepting a talar component of an ankle prosthesis, and to therefore correctly position the implant in the anterior/posterior plane.

To this end, the interposable portion 42 of the support element 38 is located between a distal end of the tibia and a previously resected normal superior surface of the talus, and is releasably attached, via the inferior surface, to the resected normal superior surface of the talus, typically by pinning or screwing, so that the guide element 40 projects anteriorly and inferiorly. The spacing of the guide slot 46 from the anterior surface of the talus is selected by the use of a spacer (not shown). A thickness of the spacer is generally based on a preselected talus component to be used. The spacer may simply be a cutting head 56 of the cutting device.

With the jig 36 in place, a size of cutting head 56 is selected, based on the talus component to be inserted. A shaft 50 of the rotatable cutting device 52 is located in the guide slot 46, and the cutting head 56 is received between the talus and the guide element 40. The cutting device is then operated whilst moving the shaft 50 back and forth along the guide slot 46.

The orientation of the guide slot 46 causes the cutting device 52 to be guided laterally relative to the foot of the patient and, due to the angle of the guide element 40, and thus the consequential angle of attack of the cutting head 56, an angled superior anterior surface is formed on the talus contiguously with the previously formed normal superior surface. The angled superior anterior surface thus forms an obtuse included angle with a longitudinal extent of the tibia. Since the talus component to be inserted is known, the posterior surface can be extremely accurately cut using a posterior talus resecting jig , due to the accurately cut anterior surface.

The talus is thus ready to receive a complementarily angled inferior surface of a talar component with optimum placement in the anterior/posterior direction or sagittal plane.

Although the block-shaped interposable portion 42 is beneficial for maintaining a spacing between the distal end of the tibia and the superior surface of the talus, the interposable portion can be a plate instead of a block.

Furthermore, the interposable portion can be dispensed with altogether. In this case, the support element is directly attached to the patient's foot at a position which is spaced or remote from the resected normal superior surface of the talus. For example, the support element can be attached to the patient's foot anteriorly or laterally of the guide element. Additionally or alternatively, it is further envisaged that the support element can be attached, either directly or indirectly, to the patient's tibia.

Although the talus resecting jig is formed integrally as a single one-piece device, it can be formed from two or more parts. For example, a removable guide element, thus enabling selection of a guide element with a differently spaced or orientated guide slot, would enable different sizes of cutting head to be utilised, or the jig to more readily accommodate a different size of talus.

The guide slot of the guide element can extend from either side of the guide element, and it is also envisaged that the guide slot could extend from an edge of the guide element which extends transversely to the side edge.

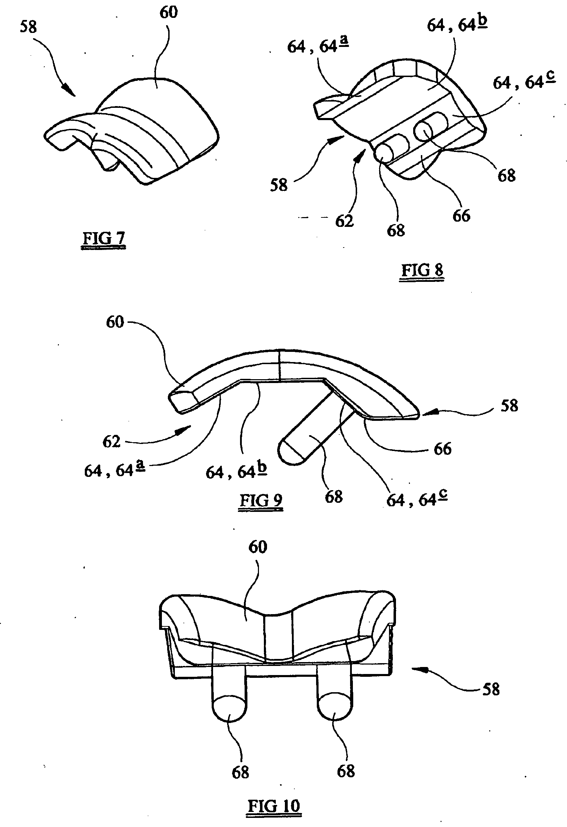

Referring now to Figures 8 to 10, there is shown a talar component 58 for an ankle prosthesis, according to the present invention, the talar component 58 comprising a superior bearing surface 60 with an articular profile for seating a complimentarily-shaped mobile talar bearing (not shown), and a concave multi-faceted inferior surface 62.

The shape of the superior surface 60 is well known, and thus will not be described in any particular detail.

The inferior surface 62 has three planar talus contacting surface portions 64 formed contiguously in an anterior to posterior direction. Each surface portion 64 has a minor dimension which extends in the anterior to posterior direction of the talar component 58, and a major dimension which is greater than the minor dimension and which extends across or substantially across the lateral extent of the talar component 58, normal to the anterior-posterior direction.

The longitudinal extents of the surface portions 64 extend in parallel or substantially parallel with each other in the lateral direction of the talar component 58.

The posterior surface portion 64a forms an obtuse included angle, typically in the range of 140° to 160° and preferably 150°, with the middle surface portion 64b in an anterior/posterior direction of the talar component 58, so that, with the middle surface portion 64b horizontal or substantially horizontal, the posterior surface portion 64a extends inferiorly or downwards. This angle allows easy resection of the posterior slope through the joint gap and permits a relatively thin bearing to be used without loss of excessive bone stock.

The anterior surface portion 64c also forms an obtuse included angle, typically in the range of 125° to 145° and preferably 135°, with the middle surface portion 64b in an anterior/posterior direction of the talar component 58, so that, with the middle surface portion 64b again horizontal or substantially horizontal, the anterior surface portion 64c extends inferiorly or downwards.

An anterior edge of the talar component 58 includes a laterally-extending radiused portion 66 which blends into the anterior surface portion 64c.

Two wedging bollards 68 are integrally formed on the anterior surface portion 64c. The bollards 68 are aligned in spaced parallel relationship in the lateral direction of the anterior surface portion 64c, and are spaced inwardly from side edges of the talar component 58. Each bollard 68 is around 10 millimetres in length and projects from the anterior surface portion 64c in a direction from the superior surface to the inferior surface of the talar component 58.

Each bollard 68 is cylindrical or substantially cylindrical, and has a longitudinal extent which is normal or substantially normal to the plane of the anterior surface portion 64c. The longitudinal extent of each bollard 68 is perpendicular to the anterior surface portion 64c, and the longitudinal extent in a proximal to distal direction of each bollard 68 thus diverges relative to a plane of the posterior surface portion 64a.

The talar component 58 is formed from any suitable bio-compatible material, such as cobalt chrome, stainless steel, or titanium alloy. The superior surface typically includes a titanium nitride coating or other suitable coating to increase hardness and to reduce third body abrasion. The inferior surface 62 of the talar component 58 is coated with hydroxyapatite, calcium phosphate, or any other suitable oestoconductive material, to promote bony ongrowth and long term stability.

To introduce the talar component 58 onto a talus of a patient, a superior surface of the talus is first resected. With the patient's foot in a condition with no flexion, and thus at or substantially at right angles to the longitudinal extent of the tibia, the talus is resected to provide a first surface which is normal or substantially normal to the longitudinal extent of the tibia. This normal resected surface corresponds to the middle surface portion 64b of the talar component 58, and this resection can be accomplished in a straightforward manner by, for example, using the ankle jig 10 described above.

To provide an anterior angled surface on the talus, which is contiguous with the normal resected surface and which corresponds to the anterior surface portion 64c of the talar component 58, the talus resecting jig 36 described above can be conveniently utilised. An alternative is for the surgeon to undertake the resection by eye and trial-and-error, although this is not preferable.

A posterior angled surface on the talus, which is typically contiguous with the normal resected surface and which corresponds to the posterior surface portion 64a of the talar component 58, is formed by the surgeon using the posterior talus resecting jig

A trial talar component without boards can be utilised to check the conformance of the seating of the multi-faceted inferior surface on the multi-faceted resected superior surface of the talus. Once satisfied that the talar component seats snugly on the resected talus, complimentary holes to receive the bollards are formed.

The talar component 58 is then fed onto the resected talus surface in an anterior to posterior direction, and the bollards 68 are located in their respective holes.

Due to the longitudinal extent of the wedging bollards 68 diverging from the plane of the posterior surface portion 64a of the talar component 58, wedging engagement of the talar component 58 with the resected talus is achieved, thus significantly increasing stability and engagement of the talar component 58 on the patient's talus.

Although the bollards extend in parallel with each other, they can converge or diverge to increase the wedging engagement.

The bollards are generally pin shaped, but can be of any suitable shape. For example, the bollards can be fully or partially frusto-conical, or have a non-circular lateral cross-section, such as square, rectangular or polygonal.

It is suggested that two bollards are provided. The bollards promote stability, particularly in a lateral direction of the talar component. However, one bollard or more than two bollards can be provided. The or each bollard acts as a stabilisation keel or element, as well as promoting greater fixation due to the wedging action achieved in conjunction with the posterior surface portion. Thus, providing these functions are achieved, the bollards can be block-shaped or any other suitable shape.

The or each bollard is provided on the anterior surface portion of the inferior surface of the talar component.

Furthermore, although preferably three contiguous planar surface portions are described, more than three contiguous planar surface portions which extend consecutively in an anterior to posterior direction and/or a lateral direction of the component can be provided.

It is thus possible to provide an ankle resecting jig for an ankle prosthesis which allows accurate resection of the tibia and the talus, whereby correct tension of the ankle ligaments is maintained. A method of balancing ankle ligaments during ankle prosthesis implantation, preferably uses the aforementioned ankle resecting jig.

It is further possible to provide a talus resecting jig which simplifies the formation of a superior anterior surface on a talus at an angle to a longitudinal extent of a patient's tibia. A method of forming a superior anterior surface on a talus of a patient at a non-perpendicular angle to the longitudinal extent of the tibia conveniently utilises the aforementioned talus resecting jig.

It is also possible to provide a talar component for an ankle prosthesis which provides increased stabilisation through the use of a multi-faceted inferior surface. The use of wedging bollards also advantageously permits increased fixation through wedging engagement with the talus.

The embodiments described above are given by way of examples only, and various other modifications will be apparent to persons skilled in the art without departing from the scope of the invention, as defined by the appended claims.

## Claims

1. A talar component (58) comprising a superior surface profiled to receive a talar bearing, and a concave inferior surface (62), the inferior surface (62) having three planar contiguous surface portions (64) which are angled relative to each other, each surface portion (64) extending across or substantially across a lateral extent of the talar component (58) wherein one or more wedging stabilisation elements extend from one or more of the surface portions (64) in a direction from the superior surface to the inferior surface (62) being **characterised by** the or each stabilisation element extending from only an anterior said surface portion 64) which is at or closest to the anterior edge of the component.

2. A talar component as claimed in Claim 1, wherein the included angle between adjacent surface portions (64) is obtuse.

3. A talar component as claimed in Claim 1 or Claim 2, wherein a longitudinal extent in a proximal to distal direction of the or each stabilisation element diverges relative to a posterior said surface portion (64) which is at or closest to the posterior edge of the component.

4. A talar component as claimed in anyone of Claims 1 to 3, wherein two said stabilisation elements are provided in spaced parallel relationship.

5. A talar component as claimed in Claim 4, wherein the said stabilisation elements are spaced in a lateral direction of the component.

## Patentansprüche

1. Eine Taluskomponente (58), umfassend eine obere Oberfläche, profiliert zur Aufnahme einer Talusstütze, und eine konkave untere Oberfläche (62), wobei die untere Oberfläche (62) drei planare angrenzende Oberflächenabschnitte (64) aufweist, die relativ zueinander gewinkelt sind, wobei sich jeder Oberflächenabschnitt (64) über oder im Wesentlichen über ein laterales Ausmaß der Taluskomponente (58) erstreckt, wobei sich ein oder mehrere Verkeil-Stabilisierungselemente von einem oder mehreren der Oberflächenabschnitte (64) in einer Richtung von der oberen Oberfläche zu der unteren Oberfläche (62) erstrecken, **dadurch gekennzeichnet, dass** sich das oder jedes Stabilisierungselement nur von einem anterioren Abschnitt des Oberflächenabschnitts (64) erstreckt, der sich an dem oder am nächsten zu dem anterioren Rand der Komponente befindet.

2. Eine Taluskomponente nach Anspruch 1, wobei der eingeschlossene Winkel zwischen benachbarten Oberflächenabschnitten (64) stumpf ist.

3. Eine Taluskomponente nach Anspruch 1 oder Anspruch 2, wobei ein longitudinales Ausmaß in einer proximalen nach distalen Richtung des oder jedes Stabilisierungselements relativ zu einem posterioren Abschnitt des Oberflächenabschnitts (64) divergiert, der sich an dem oder am nächsten zu dem posterioren Rand der Komponente befindet.

4. Eine Taluskomponente nach einem der Ansprüche 1 bis 3, wobei zwei der Stabilisierungselemente in einem beabstandeten parallelen Verhältnis vorgesehen sind.

5. Eine Taluskomponente nach Anspruch 4, wobei die Stabilisierungselemente in einer lateralen Richtung der Komponente beabstandet sind.

## Revendications

1. Élément astragalien (58) comprenant une surface supérieure profilée pour recevoir un support astragalien, et une surface inférieure (62) concave, la surface inférieure (62) ayant trois parties de surface (64) contiguës planes qui sont inclinées les unes par rapport aux autres, chaque partie de surface (64) s'étendant à travers ou sensiblement à travers une étendue latérale de l'élément astragalien (58), un ou plusieurs organes de stabilisation de calage s'étendant à partir d'une ou plusieurs des parties de surface (64) dans une direction allant de la surface supérieure à la surface inférieure (62), qui est **caractérisé par le fait que** le ou chaque organe de stabilisation s'étend à partir de seulement une partie de surface précitée (64) antérieure qui est au niveau ou la plus proche du bord antérieur de l'élément.

2. Élément astragalien selon la revendication 1, dans lequel l'angle inclus entre des parties de surface (64) adjacentes est obtus.

3. Élément astragalien selon la revendication 1 ou 2, dans lequel une étendue longitudinale dans une direction proximale à distale du ou de chaque organe de stabilisation diverge par rapport à une partie de surface précitée (64) postérieure qui est au niveau ou la plus proche du bord postérieur de l'élément.

4. Elément astragalien selon l'une quelconque des revendications 1 à 3, dans lequel deux organes de stabilisation précités sont disposés dans une relation parallèle espacée.

5. Élément astragalien selon la revendication 4, dans lequel lesdits organes de stabilisation sont espacés dans une direction latérale de l'élément.
